# EUROPEAN PATENT APPLICATION

(11) **EP 4 614 505 A1**
(43) Date of publication of application: **10.09.2025**
(21) Application number: 24161603.6
(22) Date of filing: 05.03.2024
(51) Int. Cl.: G16H 20/30, A61B 5/11, A61B 5/00, G16H 50/20, G16H 50/30

(54) **ATHLETIC PERFORMANCE MONITORING METHOD AND SYSTEM**

(71) Applicant: Project AP d.o.o., 1000 Ljubljana (SI)
(72) Inventor: MRGOLE, GREGOR, 1360 Vrhnika (SI)
(74) Representative: Best, Bastian

(57) **Abstract**

According to a first aspect of the present invention, it may be provided a computer-implemented athletic performance monitoring method. The method may comprise receiving input data including biomechanical measurement data captured by a sensor module of an article of footwear worn by a user engaged in an athletic activity. Furthermore, the method may include processing, preferably automatically processing, the input data using artificial intelligence to generate athletic performance information. This processing may involve generating a prompt for a generative language model based at least in part on the input data, prompting the generative language model using the prompt to obtain a reply, and generating the athletic performance information based at least in part on the reply from the generative language model. Additionally, the method may comprise causing displaying the athletic performance information to the user on a display screen of an electronic device of the user.

## Description

### TECHNICAL FIELD

The present invention relates to the field of sports technology and athletic performance analytics. More specifically, this invention pertains to a computer-implemented method and system for monitoring, analyzing, and enhancing athletic performance through advanced data processing techniques involving artificial intelligence (Al) and machine learning (ML). It encompasses interactive methods that employ sophisticated data interpretation and user feedback mechanisms that utilize a generative language model to provide real-time, personalized athletic insights and recommendations to users during and after engaging in athletic activities.

### BACKGROUND

In the field of athletic performance monitoring, it is common to employ various tools and devices to track and enhance an athlete's performance. Known systems typically involve the use of wearables, sensors, and data analysis tools to provide insights into an athlete's biomechanics, physiology, and overall athletic capabilities. These conventional systems provide valuable data that can be leveraged to improve training and optimize performance.

Despite the substantial advances in the field of athletic performance monitoring, there remains a need for further improvements and innovation, particularly in order to enhance usability, timing and value of information. Current systems often fall short in this respect, in particular in engage with users in a manner that feels intuitive and accessible while providing personalized guidance.

It is therefore a technical problem underlying the present invention to provide a method and system for athletic performance monitoring that at least partially overcomes the disadvantages of known systems.

### SUMMARY OF THE INVENTION

The problem is solved by the subject-matter defined in the independent claims. Advantageous modifications of embodiments of the invention are defined in the dependent claims as well as in the description and the figures.

According to a first aspect of the present invention, it may be provided a computer-implemented athletic performance monitoring method. The method may comprise receiving input data including biomechanical measurement data captured by a sensor module of an article of footwear worn by a user engaged in an athletic activity. Furthermore, the method may include processing, preferably automatically processing, the input data using artificial intelligence to generate athletic performance information. This processing may involve generating a prompt for a generative language model based at least in part on the input data, prompting the generative language model using the prompt to obtain a reply, and generating the athletic performance information based at least in part on the reply from the generative language model. Additionally, the method may comprise causing displaying the athletic performance information to the user on a display screen of an electronic device of the user.

The step of receiving input data may be performed via a wireless communication channel being established between the electronic device and the article of footwear, wherein the wireless communication channel may be based on Bluetooth and/or Wi-Fi, optionally wherein an synchronization routine is performed via the wireless communication channel, preferably automatically in predetermined time intervals. The biomechanical measurement data may include a time dependency, in particular a time course.

The method may be performed by a cloud platform and/or by the electronic device.

The term "receiving" may, in the context of the present invention, be understood as the act of obtaining or accepting input data transmitted from one or more sources. This may include, for instance, data captured from sensors.

The term "input data" may, in the context of the present invention, be understood as any data that is received or fed into the system for further processing. This data may include, but is not limited to, digital or analog signals, measurements, and observations collected by sensors or entered by users. In particular, the input data may specifically include biomechanical measurement data related to the user's movement, position, or physiological status during an athletic activity. Examples of biomechanical measurement data may include force applied by a foot during athletic activity, gait pattern, acceleration, or any other motion-specific data point that aids in performance analysis. It is preferred that the input data at least includes one or more of the following: pressure distribution of the athlete's feet, gait data of the athlete's feet. Based on the input data, it may be provided that an activity type is determined. A determined activity type may be used afterwards as an additional input data in order to further refine the subsequent output(s), i.e., the athletic performance information.

The term "biomechanical measurement data" may, in the context of the present invention, be understood as data that represents the mechanical aspects of biological movements. This data can be captured via various types of sensor modules, as further described below.

The term "sensor module" may, in the context of the present invention, be understood as a component or set of components capable of capturing and preferably transmitting biomechanical measurement data. Examples may include accelerometers, gyroscopes, or pressure sensors incorporated into an article of footwear.

The term "article of footwear" may, in the context of the present invention, be understood as any form of shoe that is possible to be equipped with the technology to measure and send biomechanical measurement data, in particular with a sensor module as defined above.

The term "athletic activity" may, in the context of the present invention, be understood as any physical activity that is aimed at maintaining or improving fitness. It may include activities such as running, walking, or jumping.

The term "automatically" may, in the context of the present invention, be understood as a process that functions with essentially no human intervention. For example, automatically processing may be performed through pre-determined rulesets and/or the use of artificial intelligence.

The term "athletic performance information" may, in the context of the present invention, be understood as processed data that provides insights into the user's athletic performance, potentially including aspects like efficiency, technique, or risk of injury derived from raw biomechanical data. It is preferred that the athletic performance information includes one or more of the following: pressure distribution of the athlete's feet, gait analysis, activity type. The activity type may include an automatically determined type of activity the athlete is performing and/or has performed.

The term "prompt" may, in the context of the present invention, be understood as an input sequence and/or instruction to an artificial intelligence, particularly a generative language model, that triggers the generation of an output or corresponding response. Generating a prompt may be performed based on user input and/or based on a set of rules and/or based on a pre-processing artificial intelligence model performing prompt adaptation based on user input and/or based on user preferences.

The term "generative language model" may refer to a machine learning model that is capable of generating text or other language-based output by responding to a given piece of input text, known as a 'prompt.' Examples of generative language models may include models like OpenAl's GPT (Generative Pre-trained Transformer) series or other similar neural network-based technologies.

The term "electronic device" may, in the context of the present invention, be understood as a device capable of processing, displaying, and/or communicating information. Specific examples can include smartphones, tablets, smartwatches, or computers.

The term "displaying" generally refers to the act of presenting visual information by the electronic device. It may pertain to the process of visually representing the generated athletic performance information on a display screen, such as that of a smartphone, tablet, smartwatch, or any other suitable electronic device capable of graphical output that the user possesses or interacts with.

The integration of a sensor module into the user's footwear facilitates enhanced accuracy and precision in the measurement of athletic performance. This high-resolution biomechanics data serves as the foundation for meticulous performance analysis, allowing for nuanced insights into the user's physical activities. Further, a primary advantage of the invention lies in its ability to deliver real-time feedback, which is crucial for athletes who seek to make immediate adjustments to their technique. This continual feedback loop can lead to expedited improvements in performance, thereby enhancing the overall efficacy of their training regimen.

With the aid of sophisticated artificial intelligence processing, the method provides personalized analysis of the user's performance. This bespoke feedback is tailored to the user's unique athletic behavior and patterns, thereby ensuring that the insights are highly relevant and actionable for the individual athlete. The implementation of a generative language model within the processing enables the system to communicate complex performance insights in a manner that is more interactive and understandable for the user. This approach transforms raw data into intelligible and engaging information, which is particularly important for facilitating user comprehension and application of the feedback.

Athletes are likely to find increased motivation and engagement through this interactive and technologically advanced approach to performance monitoring. The state-of-the-art method not only serves as a tool for assessment but also as an innovative means of engaging with one's own athletic data, ultimately fostering an enriched connection with the training process. One of the salient benefits is the system's ability to promptly identify and address inefficiencies or errors in an athlete's technique. This timely correction capability is instrumental in reducing the risk of injury and ensuring safer athletic practice, thereby supporting long-term athletic health and development.

The invention's reliance on wearable technology is another significant advantage, as it ensures that data collection is integrated seamlessly into the athlete's regular athletic activities. This convenience allows for unencumbered data capture, eliminating the need for intrusive or cumbersome equipment that could otherwise impede the user's natural performance.

It may be provided that the generative language model is a large language model (LLM) trained on a text corpus comprising general athletic performance knowledge.

The term "large language model (LLM)" may be understood as a subset of machine learning models known for their vast parameter counts and extensive training on broad datasets. These models are designed to generate, understand, and converse in human language with a high degree of proficiency. An LLM's comprehensive training enables it to encapsulate a wide array of human knowledge, thereby allowing it to simulate human-like understanding and responses in various domains.

In particular, the "general athletic performance knowledge" mentioned as part of the training corpus for the LLM may be understood as knowledge that encompasses foundational concepts, strategies, and best practices common across various sports and athletic disciplines. This generalized knowledge does not pertain to the idiosyncrasies or specific performance metrics of the individual athlete using the sensor-enabled footwear but instead is derived from a macro view of athletics. It could encompass widely accepted training methods, injury prevention techniques, and performance optimization strategies that are applicable across a spectrum of athletic activities.

Considering such a comprehensive scope of training, the LLM in the context of the present invention is capable of generating fitness tips and insights with the depth that transcends individual experiences or specialized expertise. By utilizing an LLM trained in this manner, the computer-implemented method can provide users with advice and performance feedback that reflects a broad understanding of athletic performance. This allows athletes to receive advice that, while not personalized, is highly educated and refined, similar to best practices that would be suggested by a human expert in athletic training. This aspect of the method, employing a large language model trained on extensive text corpora of general athletic knowledge, further reinforces the utility and robustness of the performance monitoring tool provided.

It may be provided that the biomechanical measurement data includes plantar foot pressure distribution data and/or 3D pressure distribution data and/or gait data and/or posture data.

The term "plantar foot pressure distribution data" may, in the context of the present invention, be understood as information pertaining to the distribution of pressure across the sole of the foot during various phases of an athlete's stride. This data is vital in understanding the foot's interaction with the ground and can reveal insights into alignment, stride efficiency, and potential areas at risk for injury.

The term "3D pressure distribution data" may, in the context of the present invention, be understood to encompass information regarding the distribution of pressure in the horizontal direction as well as the vertical direction, thereby offering a three-dimensional view of the pressure exerted by the foot. This can provide a comprehensive analysis of the forces involved in the athlete's movement.

The term "gait data" may be defined as biomechanical measurements related to the manner or pattern of an individual's walking or running. Such metrics are instrumental in assessing rhythm, balance, symmetry, and the overall efficiency of movement, which are critical factors for athletic performance.

The term "posture data" implies metrics related to the alignment and positioning of the body as a whole. Since posture greatly influences biomechanics, such data can be crucial in the assessment of an athlete's form and the likelihood of developing certain types of injuries due to misalignment or compensatory movements.

By factoring in diverse aspects such as plantar foot pressure, three-dimensional force distribution, gait patterns, and overall posture, the system can offer a holistic view of an athlete's movement and biomechanical dynamics. The comprehensive nature of this data, in tandem with the advanced processing capabilities described in the previous claims, supports a multifaceted approach to enhancing athletic performance, precision in technique correction, and injury prevention strategies, all tailored to the intricacies of individual biomechanical patterns.

It may be provided that the input data includes external data received from at least one source other than the article of footwear. The external data comprises one or more of: long-term athlete performance data, such as training intensity, recovery periods, and/or physical responses over time; secondary measurement data captured by a second sensor module, preferably of an equipment, a wearable device, and/or a sensor embedded in a garment worn by the individual; diet data; sleep pattern data; stress level data.

The term "long-term athlete performance data" may, in the context of the present invention, be understood as cumulative information reflecting the athlete's performance over an extended period. This may include data on training intensity, recovery periods, and/or physical responses, which may provide a comprehensive view of the athlete's development, stamina, and/or adaptation to various training regimens over time.

The term "secondary measurement data" refers to data captured by an additional sensor module that can augment the primary data captured by the sensor module of the article of footwear. This could relate to equipment used during athletic activities, other wearable devices, or sensors integrated into garments, which can offer multidimensional insights into the athlete's physical state and actions.

The term "diet data" may include information related to the athlete's nutritional intake, which may impact athletic performance, recovery, and/or overall well-being. This data can help tailor dietary recommendations to maximize the athlete's performance outcomes.

The term "sleep pattern data" is understood to pertain to information regarding the athlete's sleep duration and/or quality. Proper rest is important for optimal performance and recovery, and tracking sleep may offer valuable insights for adjustments in training and rest schedules.

The term "stress level data" relates to metrics that provide an insight into the athlete's psychological or physiological stress. This may include heart rate variability, hormone levels, and/or self-reported stress scales, which are imperative for understanding the athlete's readiness and capacity for training and competition.

The incorporation of such extensive external data into the performance monitoring method extends its analytical capabilities. This enables a holistic overview of the athlete's lifestyle, habits, and external factors that all contribute to performance. By integrating this multifaceted data, the method can provide actionable insights that are not only based on immediate biomechanical feedback but also encompass long-term health and performance optimization. Factors such as training habits, recovery adequacy, diet, sleep quality, and stress levels are all crucial elements that, when monitored and analyzed correctly, can significantly enhance the efficacy of the athlete's training protocol and contribute to achieving peak performance.

It may be provided that the athletic performance information comprises intra-activity athletic performance information; and wherein the step of displaying the athletic performance information comprises displaying the intra-activity athletic performance information while the individual performs the athletic activity.

Providing real-time feedback to the user based on the processed biomechanical measurement data may be performed automatically during a physical activity and/or upon receiving a triggering input of the user.

The term "intra-activity athletic performance information" may, in the context of the present invention, be understood to refer to performance data dynamically generated during the course of an athletic activity. This data may provide immediate insights and metrics that reflect the individual's performance in real-time, such as pace, power output, form consistency, and other critical parameters that can offer guidance or corrective feedback as the activity is ongoing.

The action of "displaying the intra-activity athletic performance information" necessitates an interface or device that can present the real-time performance data to the individual while they are engaged in the athletic activity. This could involve heads-up displays, smartwatches, mobile devices, or even augmented reality systems that allow the user to receive and potentially interact with performance data without interrupting the flow of their activity.

An advantage of this provision lies in the immediacy of the performance feedback. By supplying intra-activity athletic performance information, the method allows athletes to make on-the-fly adjustments to their technique, effort, or overall strategy, which could positively influence the outcome of their training or competitive endeavors. This immediate feedback loop serves to enhance the effectiveness of the athletic activity by allowing athletes to correct, hone, or optimize their performance as actions are being performed, which is a substantial enhancement over post-activity reviews that cannot influence the performance already rendered.

If such information is provided in accordance to the athletic activity, the method ensures that the athlete is not unduly distracted by the need to analyze data, thereby maintaining the integrity and flow of the workout or competition. This seamless integration of data access and athletic performance embodies a user-centric approach where technology enhances athletic experiences without becoming an invasive element.

It may be provided that the athletic performance information comprises post-activity athletic performance information; and wherein the step of displaying the athletic performance information comprises displaying the post-activity athletic performance information after the individual has finished the athletic activity.

The term "post-activity athletic performance information" may, in the context of the present invention, be understood as performance data that is analyzed and presented after the completion of the athletic activity. This may include comprehensive metrics based on the entirety of the activity, such as total distance covered, average and/or peak performance statistics, technique analysis, and/or progression over time. It may offer a retrospective review that enables reflection and long-term planning for improvement.

"Displaying the post-activity athletic performance information" involves presenting the analytical results to the individual once they have ceased the active portion of their workout, practice, or competitive event. The display can be on various devices including, but not limited to, smartphones, computers, tablets, and/or integrated systems in fitness equipment, tailored to provide the aspects of the athletic performance at the user's convenience.

By offering post-activity athletic performance information, the method enables athletes to engage in thorough review sessions, where they can assess their performance at leisure and with a greater capacity for absorption and contemplation. This review can play a critical role in identifying trends, acknowledging accomplishments, setting future goals, and developing strategies for ongoing improvement.

Further, post-activity feedback is instrumental in planning recovery activities and adjusting future training sessions based on past performances. It may further provide a valuable record-keeping tool that can be referenced over time to monitor changes and improvements.

It may be provided that the athletic performance information comprises one or more of the following: athlete performance trend information; a predicted future athlete performance; a training program; a suggested adjustment of a training program.

The term "athlete performance trend information" may, in the context of the present invention, be understood to encompass data that identifies patterns and/or directions in the athlete's performance over time. This may include improvements in speed, endurance, precision in technique, and/or other metrics that show progress or highlight areas needing attention.

The expression "a predicted future athlete performance" may refer to a forecasted performance outcome based on historical data and performance metrics. Algorithms and artificial intelligence may analyze the collected data to project future performance levels, considering various parameters such as past training loads, improvements, and external factors like rest and diet.

The term "a training program" refers to a structured set of athletic activities and/or regimens crafted to accomplish specific fitness or skill goals. Such a program is typically designed based on the individual's current performance data, abilities, needs, and overall athletic objectives.

The expression "a suggested adjustment of a training program" implies a modification or refinement of an existing training regimen. Adjustments are often recommended based on an analysis of collected data to optimize training effectiveness, enhance performance gains, prevent overtraining, and reduce the risk of injury.

These aspects of the athletic performance information provide a comprehensive tool for athletes aiming to achieve continuous improvement. Firstly, trend information acts as a guide for understanding how an athlete progresses over time, revealing successful aspects of training as well as areas that may be impeding progress. With the predictive capabilities, athletes and coaches can set realistic, data-informed goals and adjust expectations based on scientifically-grounded forecasts. The development or modification of a training program driven by this performance information ensures that the athlete's regimen remains responsive to changes in performance dynamics, health, and personal growth, thus fostering an optimal training environment. These insights collectively serve to empower athletes and coaches with a data-rich, adaptive approach to training that maximizes potential and sustainability in athletic pursuits.

It may be provided that the athletic performance information comprises a predictive forecasting of at least one injury risk. Optionally, the athletic performance information may comprise a real-time alert and/or a recommended preventive action, in particular specific exercises and/or changes in technique and/or rest periods, being generated based on the predictive forecasting. Additionally, optionally, unusual foot pressure patterns and/or asymmetrical movements and/or excessive joint stress may be detected and included in the predictive forecasting.

The term "predictive forecasting of at least one injury risk" pertains to the use of collected data and analytical models to estimate the probability of an athlete incurring an injury. This forecasting can take into account various factors such as the intensity of training, recovery status, biomechanics, and historical injury data to provide a data-driven risk assessment.

When the athletic performance information "comprises a real-time alert," it indicates an immediate notification issued to the athlete, which warns them of a detected potential injury risk during their activity. This real-time aspect is vital as it provides the opportunity for immediate action to mitigate or avoid the risk.

"A recommended preventive action" may be understood as an actionable advice derived from the predictive injury risk analysis. This advice may include, but is not limited to, specific exercises intended to strengthen vulnerable areas, modifications in technique to alleviate undue strain and/or stress, and/or guidance on the optimal duration and scheduling of rest periods to ensure adequate recovery and prevent overuse injuries.

Further, the predictive forecasting may incorporate the detection of "unusual foot pressure patterns," which would be indicative of potentially harmful gait or foot strike anomalies. Similarly, detection of "asymmetrical movements" can reveal muscle imbalances or alignment issues that may lead to injury if unaddressed. Additionally, consideration of "excessive joint stress" entails recognizing when the amount of force or load placed on joints during athletic activities could surpass safe thresholds and pose risks to the athlete's musculoskeletal health.

The capability to predict injury risks and respond effectively is of paramount importance in sports science and athlete management. By proactively identifying potential injury risk factors and implementing preventive actions, the computer-implemented method significantly contributes to athlete safety and longevity in sports. This feature advances the conventional understanding of performance monitoring by integrating holistic health management into the paradigm, making it a critical tool for athletes of all levels who seek to train effectively while minimizing injury risks.

It may be provided that the athletic performance information comprises rehabilitative advice to the user for recovering from injuries. Preferably based on the biomechanical measurement data and obtained injury information, the rehabilitative advice may include tailored rehabilitation exercises and/or a blacklist of particularly hazardous exercises and/or a whitelist of recommended exercises and/or rest period recommendations. Optionally, a rehabilitative progress of the user is taken into account based on obtained rehabilitative progress information.

The term "rehabilitative advice" in the context of the present invention refers to guidance provided to an athlete for the purpose of recovering from injuries. This advice is typically informed by detailed analyses of an athlete's specific condition and may be tailored to the individual's needs and history to optimize recovery outcomes.

When the rehabilitative advice includes "tailored rehabilitation exercises," it signifies that the exercises are customized to meet the specific rehabilitative requirements of the user's injury, with consideration for their unique biomechanical data. This approach ensures that the user engages in activities that facilitate recovery while mitigating the risk of further injury.

A "blacklist of particularly hazardous exercises" may be understood as a list of activities that the user should avoid due to the potential risk with regard to an injury. Conversely, a "whitelist of recommended exercises" indicates a list of activities deemed beneficial for a user's injury rehabilitation, based on the analysis of their condition and recovery needs.

The term "rest period recommendations" indicates the method's capability to offer guidance on the optimal amounts and scheduling of rest to help in the healing process, ensure that the user does not over-exert the injured area, and prevent complications during recovery.

The inclusion of an option where "a rehabilitative progress of the user is taken into account" refers to the method's adaptability to the user's ongoing recovery status. By incorporating obtained rehabilitative progress information, the method can continuously tailor the rehabilitative advice to match the user's evolving condition, which may include modulating exercise intensity, progression to more advanced exercises, or further rest as necessary.

The method, including the above-mentioned features, extend beyond performance monitoring and into the realm of injury management and rehabilitation support. By delivering personalized rehabilitative advice, the method plays an essential role in assisting athletes through recovery protocols that are informed by precise biomechanical data and the individual's recovery trajectory. This personalized, data-driven guidance not only helps to ensure a safe and productive rehabilitation process but also aids in the strategic return to peak performance post-injury.

It may be provided that the method further comprises generating commands, based on the step of processing, causing an adjustment of the user interaction interface, and/or causing an adjustment of the biomechanical measurement data, and/or causing an adjustment of sensor settings of the sensor module.

An "adjustment of the user interaction interface" refers to the customization or modification to how the user interfaces with the system - which could be through a graphical interface, auditory cues, haptic feedback, an/or other forms of interaction - to better suit the user's preferences, improve usability, and/or enhance the presentation of athletic performance information.

The "adjustment of the biomechanical measurement data" may include, e.g., recalibrate, refine, or modify how the biomechanical data is collected, interpreted, and/or utilized. This could lead to more accurate data capture or improved relevancy of the data.

Causing "an adjustment of sensor settings of the sensor module" entails altering the parameters or conditions under which the sensor module operates to tailor its function to more precisely match the real-time performance or condition of the user. This could include changing the sensitivity, sampling rate, and/or operational mode of the sensors to improve data accuracy or to compensate for changes in the user's activity or environment.

The potential to automatically adjust the user interface and sensor configurations ensures a more personalized and user-centric experience, optimizing the interaction between the athlete and the performance monitoring system. By enabling the system to adapt in this manner, it ensures that the data collection and user feedback mechanisms are continually refined, fostering an environment of ongoing enhancement and ensuring that athletes receive the most pertinent and actionable information for their performance improvement and injury prevention efforts.

It may be provided that the method further comprises providing a chat user interface to the user to interact with the generative language model, wherein the input data further comprises chat user input received from the user via the chat user interface.

The "chat user interface" may be understood as a platform, interface, application on the electronic device, and/or a medium that facilitates a conversational exchange between the user and, e.g., a generative language model. It is typically designed to allow the user to input text-based messages and receive responses, simulating the experience of a conversation with a generative language model. It may be designed to interpret and respond intelligently to user input.

The phrase "chat user input" refers to any textual data, queries, feedback, and/or commands that the user inputs into the chat user interface. This may include questions about performance data, requests for advice or explanations, or any other form of communication intended to trigger actions by the system.

The introduction of a chat user interface represents an innovative step in enhancing the user experience by providing an interactive and natural way for users to engage with the artificial intelligence of the system. By including the ability to receive chat user input within the input data, the method is empowered to consider the user's direct queries or concerns as part of the data processing cycle.

This highlights the interactive dimension of the method, which facilitates a two-way dialogue between the athlete and the system. Through this chat feature, users are not passive recipients of performance information; instead, they actively participate in the exploration and analysis of their data by conversing with the generative language model. This interactive approach significantly enhances the accessibility of complex performance analytics and allows for a more tailored and responsive user experience. By incorporating user feedback and inquiries in real-time, the system can provide more meaningful and personalized advice, fostering a deeper engagement with the athlete's performance and health.

It may be provided that the generative language model provides interactive engagement with the user through the chat user interface. In particular, being capable of receiving one or more subsequent user inputs, preferably in plain text format, and of providing further personalized advice and responses in response to the one or more subsequent user inputs.

"Interactive engagement with the user" facilitates a dynamic exchange where the model adapts its responses to the flow of dialogue, making the interaction resemble that with a human expert or coach. "Receiving one or more subsequent user inputs" indicates the method's ability to process and understand follow-up questions or comments from the user within the context of the ongoing conversation. It reflects the model's sophistication in maintaining a coherent and continuous interaction, which is fundamental for nuanced and detailed exchanges.

Due to the interaction being "in plain text format" underscores the user-friendly nature of the interface, enabling users to communicate with the system in everyday language without the need for special commands or technical knowledge. This encourages broader use and accessibility across a diverse user base, regardless of their technical acumen. Thus, usability is enhanced.

By supporting a series of interactions through plain text conversations, users can delve deeper into topics of interest, ask clarifying questions, and receive more thorough and personalized advice. The interactive and iterative nature of this engagement enriches the value of the performance data, turning it into a resource that users can draw upon to gain a comprehensive understanding of their athletic status and goals. The capability to handle an ongoing, context-aware conversation via the chat interface positions the system as an adaptive and intuitive coaching tool that adds significant depth to the athlete's training and recovery ecosystem.

According to a second aspect of the present invention, a data processing apparatus may be provided. The apparatus may be configured to perform a method according to the first aspect of the present invention.

The term "data processing apparatus" in the context of the present invention may be understood as a device, system, or means equipped with the necessary hardware and software components to execute the specified athletic performance monitoring methods. This could include a personal computer, server, mobile device, wearable tech, or a specialized unit designed to work in concert with sensor modules and other data sources, in particular arranged in the article of footwear.

The apparatus is specially designed or programmed to execute the steps outlined in the first aspect of the present invention. This may include, among others, receiving and handling various forms of data such as biomechanical measurement data, external data, and chat user input, processing this data using artificial intelligence and generative language models, displaying performance information, and interacting with users for both performance and rehabilitative advice.

According to a third aspect of the present invention, an interactive fitness support system may be provided. The system may include at least one data processing apparatus and an article of footwear having a sensor module, and preferably at least a cloud platform. The system is configured to perform a method according to the first aspect of the present invention. Optionally, the sensor module includes one or more pressure cells and/or one or more Inertial Measurement Units (IMUs) and/or one or more force-sensing resistors.

The term "interactive fitness support system" may be understood in the context of the present invention as a complex of interconnected elements that work together to provide an integrated solution for athletic performance monitoring and enhancement. The system facilitates interaction between the user and the technological components to support fitness activities and recovery processes.

According to a fourth aspect of the present invention, it may be provided a computer program or a computer-readable medium having stored thereon a computer program. The computer program may comprise instructions which, when the program is executed by a computer, preferably by a data processing apparatus according to the second aspect of the present invention, more preferably by a system according to the third aspect of the present invention, cause the same to carry out a method according to the first aspect of the present invention.

The term "computer program" may, in this context, be understood to encompass a set of encoded information or computer instructions designed to perform a specific task outlined in the previously referenced method claims. The program empowers computing devices to enact complex processes that facilitate the collection, processing, and analysis of athletic performance data as well as interaction with users for performance feedback and rehabilitative support.

A "computer-readable medium" refers to any data storage device that can retain and store instructions for use by an instruction execution device - such as a computer or a data processing apparatus. Examples of such media include, but are not limited to, magnetic disks (such as hard drives and floppy disks), optical discs (such as CDs and DVDs), solid-state drives (SSDs), and USB flash drives.

All features, technical implementation details and advantages described with respect to any one of the aspects of the present invention described herein are self-evidently mutatis mutandis applicable for any one of the other aspects of the present invention and vice versa.

### BRIEF DESCRIPTION OF THE DRAWINGS

The disclosure may be better understood by reference to the following drawings:
- Figure 1a: shows a component diagram according to embodiments of the present invention.
- Figure 1b: shows a detailed schematical view of an article of footwear according to embodiments of the present invention.
- Figure 2: shows a first flowchart including steps according to embodiments of the present invention.
- Figure 3: shows a second flowchart including steps according to embodiments of the present invention.
- Figure 4: shows an activity flowchart according to embodiments of the present invention.
- Figures 5-7: show aspects of a personalized dashboard and a virtual coach integration according to embodiments of the present invention.

### DETAILED DESCRIPTION

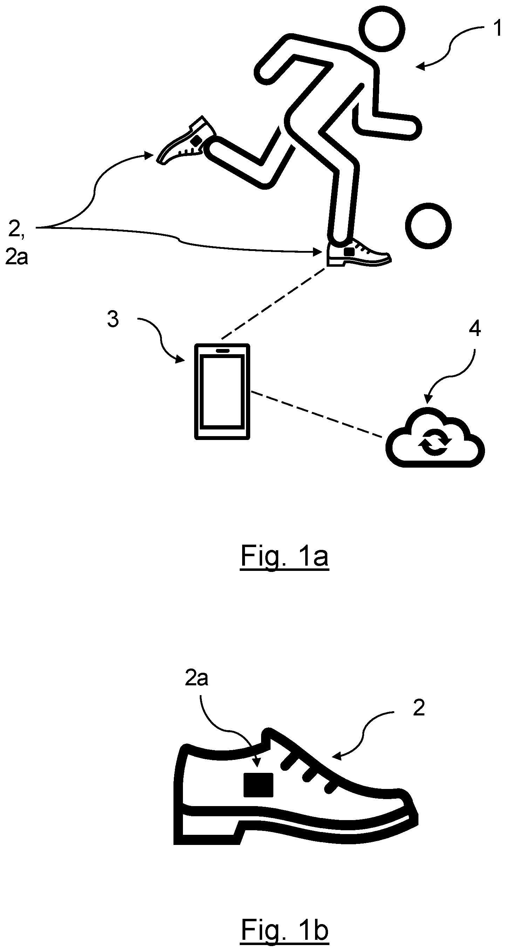

Figure 1a shows a component diagram according to embodiments of the present invention. In embodiments of the present invention, it may be provided a computer-implemented athletic performance monitoring method 100. The method 100 may comprise receiving 101 input data including biomechanical measurement data captured by a sensor module 2a of an article of footwear 2 worn by a user 1 engaged in an athletic activity. Furthermore, the method 100 may include processing 102, preferably automatically processing, the input data using artificial intelligence to generate athletic performance information. This processing may involve generating 102a a prompt for a generative language model based at least in part on the input data, prompting 102b the generative language model using the prompt to obtain a reply, and generating 102c the athletic performance information based at least in part on the reply from the generative language model. Additionally, the method 100 may comprise causing displaying 103 the athletic performance information to the user 1 on a display screen of an electronic device 3 of the user. The method 100 may be performed by a cloud platform 4 and/or by the electronic device 3.

The step of receiving input data may be performed via a wireless communication channel being established between the electronic device 3 and the article of footwear 2, wherein the wireless communication channel may be based on Bluetooth and/or Wi-Fi, optionally wherein an synchronization routine is performed via the wireless communication channel, preferably automatically in predetermined time intervals. The biomechanical measurement data may include a time dependency, in particular a time course.

Figure 1b is a detailed view of the article of footwear 2 including a sensor module 2a. The sensor module 2a may be capable of capturing and preferably transmitting biomechanical measurement data. Examples may include accelerometers, gyroscopes, or pressure sensors incorporated into the article of footwear 2. Further comprehensive examples are given below.

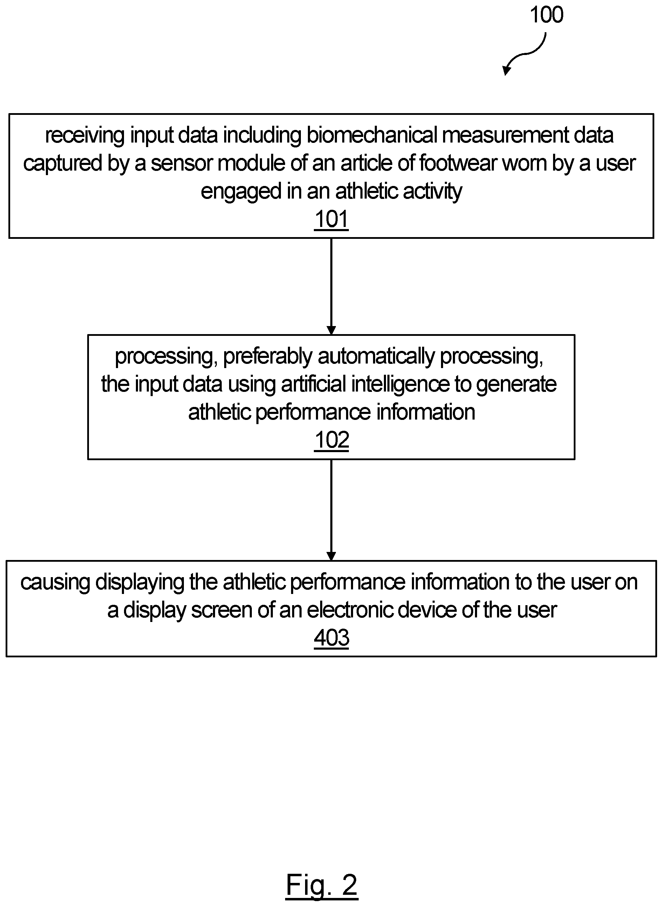

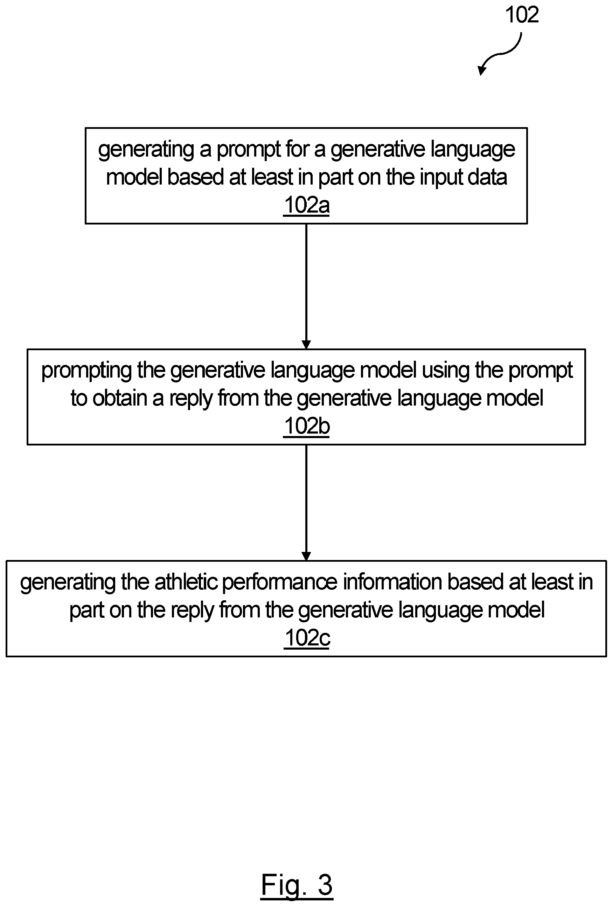

Figure 2 shows a flowchart including steps 101, 102 and 103 of the present invention. Figure 3 shows a flowchart including sub-steps 102a, 102b and 102c of step 102.

In the following, the present invention is described in slightly different words. For example, the term "article of footwear" may be replaced by the term "shoe" of "smart shoe" and vice versa. Further, the terms "user" and "athlete" may be used as synonyms. The skilled person understands any features, terms and expressions listed and described in the context of the present invention, in particular belonging to respective parts described in the summary section above.

For collecting the input data, the article of footwear may comprise a IEE ActiSense Smart Footwear Sensing Solution. This sensor solution may be part of the sensor module of the article of footwear being capable of obtaining biomechanical measurement data. It may use advanced sensing technology to capture valuable data regarding foot pressure distribution during various activities such as specific exercise execution, running, or jumping. This data may be crucial for athletes, offering insights for performance enhancement, injury prevention, and health monitoring.

The IEE Sensing System (IEE ActiSense Smart Footwear Sensing Solution) may include:
- Plantar pressure distribution,
- Gait and posture analysis,
- Stride length and cadence (in some applications), and/or
- High-pressure area identification.

Alternatively or additionally, preferably additionally, a Force Sensing Resistor Sensing System (FSR Sensing System) may be provided, in particular in the article of footwear. This may include obtaining biomechanical measurement data like:
- Normal and shear forces,
- 3D force distribution,
- Force range measurement (e.g. from 0.1N to 140N), and/or
- Pressure sensitivity at various points.

A combination of the IEE Sensing System and the FSR Sensing System is preferred. Combining both may provide a comprehensive solution for your high-end sports shoe design which may advantageously facilitate:
- Enhanced Data Collection: Combining the plantar pressure data from the IEE system with the normal and shear force measurements of the FSR system would give a more complete understanding of foot dynamics.
- Improved Performance Analysis: This combination could lead to more accurate insights into athletic performance, biomechanics, and gait patterns.
- Effective Injury Prevention: The broad range of data could be instrumental in identifying potential injury risks and designing preventive measures.
- Comprehensive Health Monitoring: The integrated system would offer detailed feedback on foot health, useful for both athletes and medical professionals.

Comparing using the lEE Sensing System alone with using it together with the FSR Sensing System, the following can be noted:
With lEE Sensing System alone tracking, e.g., a sprinting session on a track, the collected data may include plantar pressure distribution during each foot strike, stride length, and cadence. The analysis may include identifying areas of high pressure, indicating potential stress points; analyze symmetry in foot strikes for balance and stride efficiency.

Adding the FSR Sensing System enhances the value of the output by the additional measurement of shear forces and 3D force distribution. This may result in an enhanced analysis where it may be possible to also assess the lateral and forward/backward forces exerted during each foot strike. This data may be important for understanding the dynamic foot movement, especially during rapid directional changes in sprinting. The resulting insight is a more complete biomechanical analysis where it may be possible to identify not just where the pressure is concentrated, but also how the foot interacts with the shoe and ground in different phases of running, offering deeper insights for performance optimization and injury prevention.

Experimental examples for each key area, illustrating how data from the lEE and FSR Sensor Systems can be utilized are given as follows:
Strength Training:
   - IEE: Measures plantar pressure during a deadlift to assess balance.
   - FSR: Adds data on force distribution between the heel and forefoot, aiding in form correction.
Endurance Training:
   - IEE: Tracks pressure changes during a long-distance run to monitor fatigue.
   - FSR: Provides additional data on shear forces, indicating shifts in gait due to fatigue.
Flexibility and Recovery:
   - lEE: Assess foot pressure during yoga poses for recovery analysis.
   - FSR: Adds insights into force application during stretches, enhancing recovery strategies.
Speed and Agility Training:
   - lEE: Analyzes plantar pressure during sprints for foot strike patterns.
   - FSR: Offers detailed force data during rapid directional changes, refining agility drills.
Balance and Coordination:
   - IEE: Measures pressure distribution in balance exercises like one-legged stands.
   - FSR: Adds force dynamics analysis, improving balance and coordination training.
Sport-Specific Skills:
   - IEE: Tracks pressure points during sport-specific movements like a basketball pivot.
   - FSR: Provides force data during these movements, enhancing skill development.
Nutrition and Hydration:
   - Indirect benefit from both systems through data on training intensity and recovery needs.
Mental Training:
   - Objective data from both systems aids in setting measurable goals and tracking progress, boosting mental resilience and focus.

The data handling between the sensor module and an electronic device, in particular a data processing apparatus performing, e.g., a method according to the first aspect of the present invention may include:
- Data Collection: The system may employ thin, non-intrusive printed electronics, featuring high dynamic, new-generation pressure cells combined with an Inertial Measurement Unit (IMU). These sensors may accurately measure and analyze gait, posture, and other biomechanical aspects.
- Data Transmission: After collection, the data may be sent to a remote receiver, a smartphone, or a computer. This is typically done via wireless communication technologies such as Bluetooth and/or Wi-Fi. The powerful onboard electronics may ensure efficient and real-time data synchronization and transmission.
- Data Processing and Al Analysis: Once the data reaches the receiving device, it may undergo processing and analysis. An AI engine (for example: openAI backbone), integrated into a dedicated app or software on the electronic device, may interpret the data in real-time. This Al component may analyze patterns, detect anomalies, and provide actionable insights. The use of AI may facilitate transforming raw sensor data into meaningful information.
- Display and Interaction: The processed information is displayed to the user through an interface on the electronic device, e.g., mobile and/or desktop application. This interface may be designed to be user-friendly, providing insights, improvement suggestions, and warnings about potential injury risks in an understandable format. Athletes and users may interact with this data to track their performance, make informed decisions about their training, take proactive steps for injury prevention and performance optimization, and provide feedback or input, which can be used to further refine the AI analysis.
- Cross-Platform Compatibility: The application may be compatible with various devices and platforms for broader accessibility.

In addition, a meaningful aspect of the present invention may be the versatility in how data is displayed and interacted with by the end user - the athlete. This interaction may occur in three primary ways:
- Real-Time Displaying: This method may allow athletes to receive immediate feedback during their activities. As the sensors in the shoe collect data, it may be processed and displayed in real-time on a connected device. This immediate feedback may be invaluable for athletes looking to make on-the-spot adjustments to their performance, technique, or strategy.
- Post-Workout Displaying: After a training session, athletes may review a comprehensive analysis of their performance. This may include detailed insights into their biomechanics, highlighting areas of strength and opportunities for improvement. This retrospective analysis may be valuable for long-term training adjustments and injury prevention.
- Interactive Engagement with the artificial intelligence: The ability for athletes to interact with an Al, which may be referred to as the 'shoe AI', is particularly advantageous. This interaction may go beyond mere data display; athletes may engage in conversations about training sessions, exercises, nutrition, and other athlete-centric topics. The AI may provide personalized advice, answer queries, and help in planning future training sessions, all aimed at advancing athletic capabilities and preventing potential injuries.

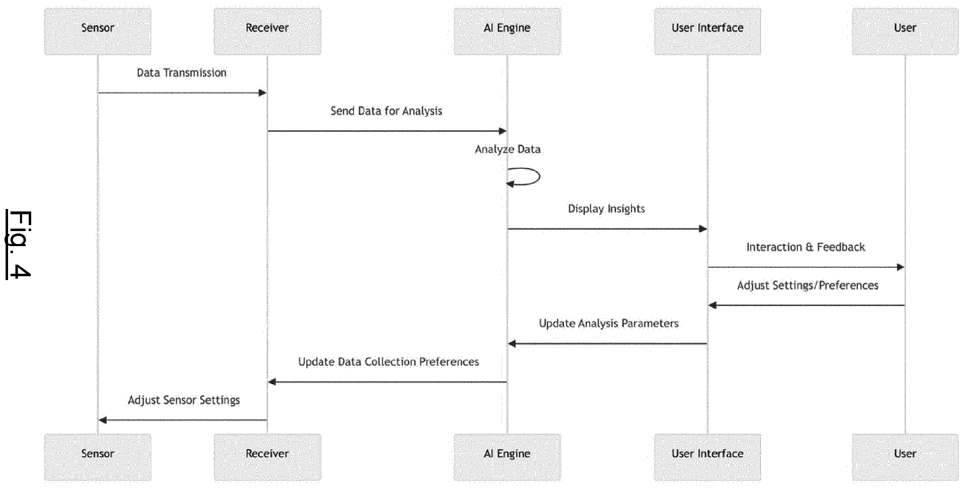

The above-described aspects are illustrated in figure 4 which is a flowchart with a particular focus of the data flow.

With regard to the AI integration, an API interfacing the OpenAI, e.g., Chat GPT, particularly preferred a Custom GPT customized via the OpenAI platform, may be provided. By this, it may be provided a suitable AI for data analysis, identifying patterns for performance improvements. Benefit to Users: Real-time insights, injury prevention, customized experience. In the following, exemplary aspects are described in this respect:
The user may take advantage of personalized training programs, e.g., including:
- Al-Driven Customization: Al algorithms may analyze the data collected by, e.g., IEE sensors to identify patterns and trends in an athlete's performance. This analysis may allow for the creation of highly personalized training programs that cater to the specific strengths and weaknesses of the user.
- Dynamic Adaptation: The Al may continuously learn from ongoing data, allowing it to adjust the training regimen in real time based on the user's progress, response to training, and evolving goals.
- Predictive Performance Modeling: Al may predict future performance outcomes based on current trends, helping to set realistic and achievable goals for the athlete.
- Exemplary training areas where an athlete may take advantage: Strength Training, Endurance Training, Flexibility and Recovery, Speed and Agility Training, Balance and Coordination, Sport-Specific Skills, Nutrition and Hydration (Indirect Benefit), Mental Training (Indirect Benefit)

The user may take advantage of personalized injury prevention strategies, e.g., including:
- Predictive Analysis: Al may predict potential injury risks by analyzing data trends such as unusual pressure patterns, asymmetrical movements, or excessive joint stress. It may identify these risks well before they manifest as actual injuries.
- Automated Alerts and Recommendations: Upon detecting a potential injury risk, the Al system may immediately alert the user and provide recommendations for preventive actions, such as specific exercises, changes in technique, or rest periods.
- Rehabilitative Support: For athletes recovering from injuries, Al may tailor rehabilitation exercises and monitor recovery progress, ensuring a safe and effective return to activity.

The user may take advantage of long-term athletic health monitoring and optimization, e.g., including:
- Holistic Health Monitoring: Al may analyze long-term data to provide insights into the athlete's overall health trajectory, considering factors like training intensity, recovery periods, and physical responses over time.
- Lifestyle Optimization: By integrating data from IEE sensors with external wearables and/or factors like diet, sleep patterns, and stress levels, Al may offer holistic lifestyle recommendations for optimal athletic performance.
- Trend Analysis and Reporting: Al may generate comprehensive reports that track the athlete's long-term progress, highlight achievements, and identify areas needing attention, supporting continuous improvement in athletic performance.

Referring back to the three primary ways of interaction, the types "Real-Time Displaying" and "Post-Workout Displaying" may be understood as an AUTO-RESPONSE routine. For such auto response, the Custom GPT may be configured based on the OpenAl GPT platform and include the following:
Exemplary instructions:
   - The GPT model may be programmed to automatically process incoming data from the IEE and FSR sensors.
   - It may handle data normalization, anomaly detection, and/or pattern recognition to ensure accurate interpretation.
   - The model may be configured to prioritize real-time data for immediate feedback and to aggregate data for comprehensive post-workout analysis.
   - Instructions also may include handling data security and privacy, ensuring that athlete's data is processed and stored securely.

Exemplary Prompts:
- For Real-Time Displaying: "Process current sensor input for immediate feedback on balance and force distribution during a sprint."
- For Post-Workout Displaying: "Compile and analyze today's workout data to provide insights on endurance improvement and potential stress points."
- These prompts indicate how the system uses sensor data to provide immediate feedback during an activity and comprehensive analysis after the workout.

This approach may allow the AI to deliver actionable insights to athletes both during and after their training sessions.

Again referring back to the three primary ways of interaction, the type "Interactive Engagement with the artificial intelligence" may be understood as an ON DEMAND RESPONSE. For such, the Custom GPT may be configured based on the OpenAl GPT platform and include the following:
Exemplary instructions:
   - The GPT model processes sensor data from the lEE and FSR systems, readying it for interactive queries.
   - It includes a conversational interface for athletes to inquire about training, nutrition, etc.
   - The model is designed to understand the context and provide personalized advice based on the sensor data and the athlete's historical performance.
   - Protocols for data privacy and personalized interaction are emphasized.
Exemplary Prompts:
   - "How was my balance during today's agility training?"
   - "Based on my last week's performance, what nutritional changes should I consider?"
   - "Plan a training session for improving my endurance based on recent data."
   - These prompts show the AI providing customized feedback and advice, engaging in a conversational manner with the athlete.

This setup may allow athletes to have meaningful interactions with the Al, gaining tailored insights and guidance for their training and overall athletic development.

Further aspects of data handling may include:
- Data Preprocessing and Integration: The sensor data may be preprocessed to match the input format of the LLM. This may involve converting numerical data into a text-based format that the LLM can understand.
- Model Training and Fine-Tuning: LLMs may be pre-trained on vast datasets, they may require additional training or fine-tuning with domain-specific data (like sports performance data) to optimize their performance for specific applications.
- Contextual Understanding and Personalization: The model may be capable of contextualizing the sensor data within the framework of athletic performance. It provides personalized feedback based on the athlete's performance data, incorporating both real-time and historical data for comprehensive analysis.

Information that may be used for processing by the Al and/or for training the Al and/or to customize the Al may include:
- Athlete personal information (gender, age, weight, height),
- Activity/Sport Type and Frequency (number of sessions per week, month)
- Specific Athlete Improvement Goals: such as speed, endurance, etc.,
- Specific Health-related goals such as: weight loss, gain muscle or strength, etc.
- Additional Health Information: Pre-existing conditions, injury history, recovery status.
- Performance Metrics: Past performance data, personal bests, training frequency, and intensity.
- Nutritional Information: Diet preferences, hydration levels, supplement use.
- Psychological Data: Stress levels, mental health status, sleep patterns.
- Environmental Data: Training conditions like climate and altitude

Based on the data from IEE and FSR sensing systems, the Al model may provide various relevant outputs for athletes, such as:
- Plantar Pressure Distribution: Analysis of pressure points during different activities.
- Gait Analysis: Information on stride length, symmetry, and efficiency.
- Force Distribution: Insights into normal and shear forces on the feet during movements.
- Balance Assessment: Data on weight distribution and balance during static and dynamic exercises.
- Foot Strike Patterns: Information on heel, midfoot, or forefoot strikes in activities like running.
- Posture Analysis: Feedback on posture alignment during various exercises.
- Fatigue Assessment: Indicators of fatigue based on changes in pressure and gait patterns.
- Injury Risk Assessment: Identification of potential injury risks based on pressure and force data.
- Performance Trends: Long-term analysis of performance changes and improvements.
- Rehabilitation Progress: Data useful for monitoring recovery from injuries.

These outputs may assist in optimizing training, preventing injuries, and improving overall athletic performance. In addition, the Al model may also provide the following outputs:
- Exercise and Training Recommendations: Tailored exercise plans based on gait analysis, foot pressure distribution, and force data, focusing on improving specific aspects of performance.
- Injury Prevention Advice: Identification of potential injury risks and advice on preventative exercises, footwear adjustments, or training modifications.
- Nutrition Guidance: Based on exercise intensity and recovery needs indicated by the sensor data, the Al can offer nutrition and hydration recommendations to optimize performance and recovery.

In the following, aspects of the User Interface (Ul) are presented.

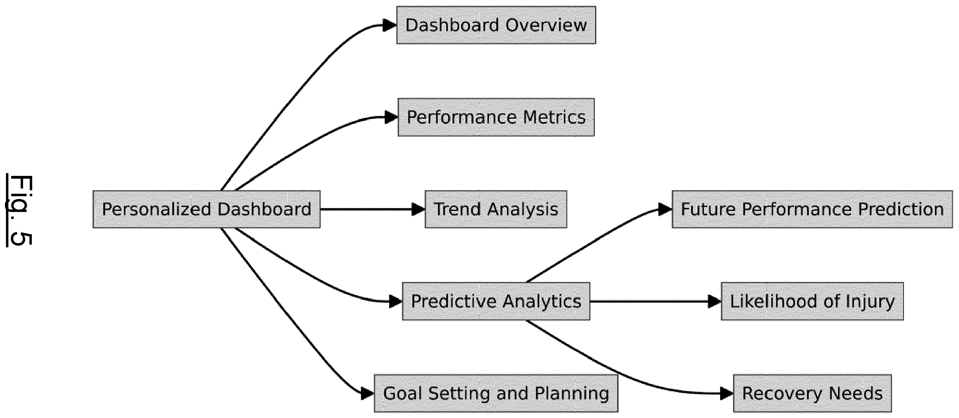

Referring to figure 5, a personalized dashboard with predictive analytics may be provided. This dashboard may compile the athlete's data into an easy-to-understand format, highlighting key performance metrics and trends. It may use Al to analyze past performance and predict future outcomes, such as potential for improvement in certain areas, likelihood of injury, or need for recovery. This predictive analysis may help in setting realistic goals and planning effective training regimes.

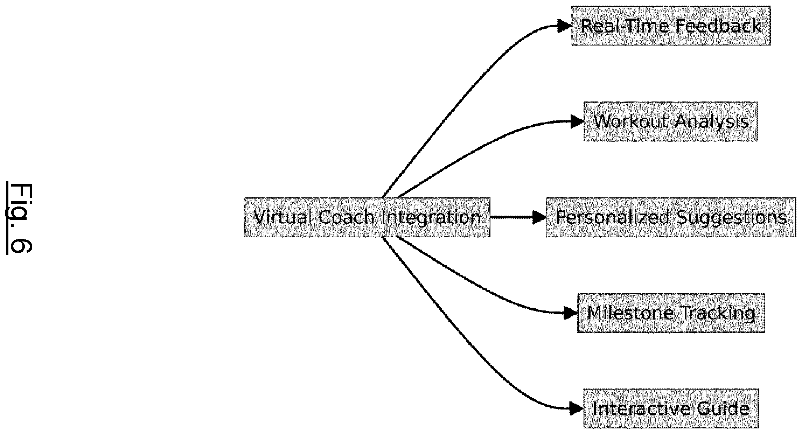

Referring to figure 6, a virtual coach integration may be provided. The virtual coach may be an Al-driven feature providing real-time feedback and advice based on the sensor data. It may offer suggestions during a workout, like adjusting form or intensity, and post-workout analysis, such as areas of improvement or congratulatory feedback on reaching new milestones. This coach may act as an interactive guide, enhancing the training experience with personalized, data-driven advice.

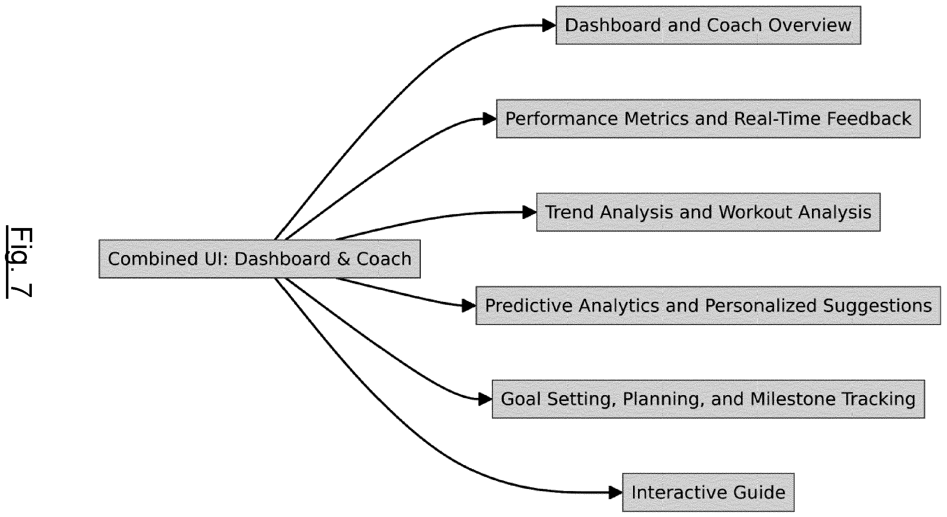

Referring to figure 7, the personalized dashboard and the virtual coach may be combined into one cohesive User Interface wireframe.

It may be provided that the input data includes external data received from at least one source other than the article of footwear. Examples may include:
Biomechanical Data:
   - IMU Sensors (Accelerometers, Gyroscopes, Magnetometers): For measuring acceleration, orientation, rotational movements, and direction.
   - Force Sensors (FSRs): To measure force exerted by muscles or on different body parts.
   - Flex Sensors: For the degree of flexion in joints.
   - Electromyography (EMG) Sensors: To assess muscle activity and fatigue.
Activity Data:
   - GPS: For tracking location and movement patterns.
   - Optical Heart Rate Monitors: For measuring heart rate through blood flow.
Health Metrics:
   - Skin Temperature Sensors: To monitor body temperature.
   - Electrodermal Activity Sensors: For measuring skin conductance, related to sweating/stress.
   - Blood Oxygen Sensors: To measure oxygen saturation levels.
   - Sweat Composition Sensors: For analyzing electrolytes, lactate, glucose, and stress markers.
Environmental and Contextual Data:
   - Ambient Temperature Sensors: To measure external temperature.
   - Humidity Sensors: To detect humidity levels.
   - UV Sensors: For UV light exposure measurement.
   - Barometric Pressure Sensors: Useful for altitude training and mountain sports.
   - Air Quality Sensors: To monitor pollutants.

These addition input data from external sources may be used, in combination with the biomechanical measurement data, for
- Personalized Feedback and Coaching: Utilizing biomechanical and activity data, the AI can offer personalized coaching tips and feedback. This may help in fine-tuning the athlete's technique and form, based on their unique movement patterns and activity levels.
- Injury Prediction and Prevention: By analyzing stress and strain patterns through sensor data, the AI may identify potential injury risks. This preemptive approach may allow athletes to adjust their training to prevent injuries, ensuring a safer and more sustainable athletic career.
- Performance Optimization: Correlating health metrics with activity data may enable the AI to optimize performance. This may involve adjusting training intensity and recovery based on heart rate, blood oxygen levels, and other health indicators, tailoring the athletic experience to the individual's physiological responses.
- Personalized Training Recommendations: The AI may tailor training programs according to both biomechanical data and environmental conditions. This holistic approach may ensure that training is not only based on the athlete's physical state but also considers external factors like temperature, humidity, and air quality, optimizing both performance and safety.
- Real-time Environmental Adjustments: The AI may provide real-time alerts about changing environmental conditions that could impact performance or health. For instance, notifying athletes of high UV exposure or poor air quality enables them to make immediate adjustments to their training regimen or location.
- Data Correlation for Enhanced Insights: By correlating environmental data with performance metrics, the AI may provide deeper insights into how different conditions affect athletic performance. This helps in understanding and adapting to the varying impacts of environmental factors on physical exertion and performance.

In the following, further aspects and details of one embodiment of the AI is given, wherein it is referred to a DeepHAR algorithm which is a deep forward neural network designed for recognizing human activities based on data collected from smart insoles in shoes. This may be integrated into the present invention.

The DeepHAR algorithm may efficiently processes sensor inputs, including pressure, force, and inertial measurements, to identify a range of activities, enhancing the capabilities of smart footwear technologies. This approach may use several advanced techniques to boost performance and address challenges such as data imbalance:
- Data Preprocessing: May include methods like data segmentation with overlapping, signal down-sampling, and a cost-sensitive re-weighting strategy to handle imbalanced datasets effectively.
- Deep Learning Model: May use a deep feed-forward architecture that, despite its relative simplicity, achieves high accuracy and performance through adequate preprocessing. It may be specifically designed to prevent overfitting and reduce computational costs, optimizing for real-time application in wearable devices.
- Class Imbalance Handling: May employ a sophisticated loss function modification to manage the uneven distribution of activity classes within the dataset, ensuring that the model remains unbiased and performs well across all activity types.

The integration of DeepHAR into the present invention's data processing process may offer a promising possible implementation. By leveraging this advanced algorithm, it is possible to provide athletes with detailed insights into their movements and activities, supporting personalized training and injury prevention strategies.

The AI Engine Layered Processing that may be used for the present invention may involve advanced algorithms and large language models (LLMs), including technologies comparable to OpenAI's GPT, to further analyze and interpret the data processed by the DeepHAR algorithm. This process may facilitate translating complex biomechanical and physiological data into actionable insights for athletes. This may include:
- Data Normalization and Anomaly Detection: Initially, the AI processes incoming sensor data by normalizing variations and identifying outliers, ensuring data consistency and reliability.
- Pattern Recognition and Insight Generation: The AI may use pattern recognition to identify trends and correlations within the data, facilitating the extraction of meaningful insights regarding performance, health, and potential injury risks.
- Real-Time and Post-Workout Feedback: The system may prioritize immediate data processing for real-time feedback during activities and aggregates data for comprehensive post-workout analysis, offering athletes a dual perspective on their performance.
- Personalized Interaction: Through a conversational Al interface, athletes can engage in dynamic interactions, receiving personalized advice, training recommendations, and nutritional guidance based on their specific data and performance history.
- Security and Privacy: Integral to the process may be the commitment to data security and privacy, ensuring that all athlete data is handled with the utmost care, respecting confidentiality and regulatory requirements.

This layered processing approach may allow for a nuanced understanding of the athlete's performance, enabling personalized enhancements to training and health management in real-time and over the long term.

Processed data may be presented to athletes through an intuitive interface that displays real-time feedback and post-workout summaries. This interface may be designed for easy interpretation of performance metrics, offering visual analytics like graphs and heatmaps. Athletes interact with this data to make immediate adjustments during activities, enhancing performance and preventing injuries. The system also may support personalized advice through the Al, enabling athletes to engage in conversations for tailored guidance and strategic planning based on their performance data.

Certain embodiments of the invention may be based on using a machine learning model or machine learning algorithm. Machine learning may refer to algorithms and statistical models that computer systems may use to perform a specific task without using explicit instructions, instead relying on models and inference. For example, in machine learning, instead of a rule-based transformation of data, a transformation of data may be used that is inferred from an analysis of historical and/or training data. For example, the content of images may be analyzed using a machine learning model or using a machine learning algorithm. In order for the machine learning model to analyze the content of an image, the machine learning model may be trained using training images as input and training content information as output. By training the machine learning model with a large number of training images and/or training sequences (e.g. words or sentences) and associated training content information (e.g. labels or annotations), the machine learning model "learns" to recognize the content of the images, so the content of images that are not included in the training data can be recognized using the machine learning model. The same principle may be used for other kinds of sensor data as well: By training a machine learning model using training sensor data and a desired output, the machine learning model "learns" a transformation between the sensor data and the output, which can be used to provide an output based on non-training sensor data provided to the machine learning model. The provided data (e.g., sensor data, meta data and/or image data) may be preprocessed to obtain a feature vector, which is used as input to the machine learning model.

Machine learning models may be trained using training input data. The examples specified above use a training method called "supervised learning". In supervised learning, the machine learning model is trained using a plurality of training samples, wherein each sample may comprise a plurality of input data values, and a plurality of desired output values, i.e., each training sample is associated with a desired output value. By specifying both training samples and desired output values, the machine learning model "learns" which output value to provide based on an input sample that is similar to the samples provided during the training. Apart from supervised learning, semi-supervised learning may be used. In semi-supervised learning, some of the training samples lack a corresponding desired output value. Supervised learning may be based on a supervised learning algorithm (e.g., a classification algorithm, a regression algorithm or a similarity learning algorithm). Classification algorithms may be used when the outputs are restricted to a limited set of values (categorical variables), i.e., the input is classified to one of the limited set of values. Regression algorithms may be used when the outputs may have any numerical value (within a range). Similarity learning algorithms may be similar to both classification and regression algorithms but are based on learning from examples using a similarity function that measures how similar or related two objects are. Apart from supervised or semi-supervised learning, unsupervised learning may be used to train the machine learning model. In unsupervised learning, (only) input data might be supplied and an unsupervised learning algorithm may be used to find structure in the input data (e.g. by grouping or clustering the input data, finding commonalities in the data). Clustering is the assignment of input data comprising a plurality of input values into subsets (clusters) so that input values within the same cluster are similar according to one or more (pre-defined) similarity criteria, while being dissimilar to input values that are included in other clusters. Reinforcement learning is a third group of machine learning algorithms that may be used to train the machine learning model. In reinforcement learning, one or more software actors (called "software agents") are trained to take actions in an environment. Based on the taken actions, a reward is calculated. Reinforcement learning is based on training the one or more software agents to choose the actions such, that the cumulative reward is increased, leading to software agents that become better at the task they are given (as evidenced by increasing rewards).

Furthermore, some techniques may be applied to some of the machine learning algorithms. For example, feature learning may be used. In other words, the machine learning model may at least partially be trained using feature learning, and/or the machine learning algorithm may comprise a feature learning component. Feature learning algorithms, which may be called representation learning algorithms, may preserve the information in their input but also transform it in a way that makes it useful, often as a pre-processing step before performing classification or predictions. Feature learning may be based on principal components analysis or cluster analysis, for example.

In some examples, anomaly detection (i.e., outlier detection) may be used, which is aimed at providing an identification of input values that raise suspicions by differing significantly from the majority of input or training data. In other words, the machine learning model may at least partially be trained using anomaly detection, and/or the machine learning algorithm may comprise an anomaly detection component.

In some examples, the machine learning algorithm may use a decision tree as a predictive model. In other words, the machine learning model may be based on a decision tree. In a decision tree, observations about an item (e.g., a set of input values) may be represented by the branches of the decision tree, and an output value corresponding to the item may be represented by the leaves of the decision tree. Decision trees may support both discrete values and continuous values as output values. If discrete values are used, the decision tree may be denoted a classification tree, if continuous values are used, the decision tree may be denoted a regression tree.

Association rules are a further technique that may be used in machine learning algorithms. In other words, the machine learning model may be based on one or more association rules. Association rules are created by identifying relationships between variables in large amounts of data. The machine learning algorithm may identify and/or utilize one or more relational rules that represent the knowledge that is derived from the data. The rules may e.g. be used to store, manipulate or apply the knowledge.

Machine learning algorithms are usually based on a machine learning model. In other words, the term "machine learning algorithm" may denote a set of instructions that may be used to create, train or use a machine learning model. The term "machine learning model" may denote a data structure and/or set of rules that represents the learned knowledge (e.g., based on the training performed by the machine learning algorithm). In embodiments, the usage of a machine learning algorithm may imply the usage of an underlying machine learning model (or of a plurality of underlying machine learning models). The usage of a machine learning model may imply that the machine learning model and/or the data structure/set of rules that is the machine learning model is trained by a machine learning algorithm.

For example, the machine learning model may be an artificial neural network (ANN). ANNs are systems that are inspired by biological neural networks, such as can be found in a retina or a brain. ANNs comprise a plurality of interconnected nodes and a plurality of connections, so-called edges, between the nodes. There are usually three types of nodes, input nodes that receiving input values, hidden nodes that are (only) connected to other nodes, and output nodes that provide output values. Each node may represent an artificial neuron. Each edge may transmit information, from one node to another. The output of a node may be defined as a (non-linear) function of its inputs (e.g., of the sum of its inputs). The inputs of a node may be used in the function based on a "weight" of the edge or of the node that provides the input. The weight of nodes and/or of edges may be adjusted in the learning process. In other words, the training of an artificial neural network may comprise adjusting the weights of the nodes and/or edges of the artificial neural network, i.e. to achieve a desired output for a given input.

Alternatively, the machine learning model may be a support vector machine, a random forest model or a gradient boosting model. Support vector machines (i.e., support vector networks) are supervised learning models with associated learning algorithms that may be used to analyze data (e.g., in classification or regression analysis). Support vector machines may be trained by providing an input with a plurality of training input values that belong to one of two categories. The support vector machine may be trained to assign a new input value to one of the two categories. Alternatively, the machine learning model may be a Bayesian network, which is a probabilistic directed acyclic graphical model. A Bayesian network may represent a set of random variables and their conditional dependencies using a directed acyclic graph. Alternatively, the machine learning model may be based on a genetic algorithm, which is a search algorithm and heuristic technique that mimics the process of natural selection.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

Some or all of the method steps may be executed by (or using) a hardware apparatus, such as a processor, a microprocessor, a programmable computer or an electronic circuit. Depending on certain implementation requirements, embodiments of the invention can be implemented in hardware or in software. The implementation can be performed using a non-transitory storage medium such as a digital storage medium, for example a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments of the invention provide a data carrier having electronically readable control signals, which are capable of cooperating with a programmable computer system, such that one of the methods described herein is performed.

Generally, embodiments of the invention can be implemented as a computer program (product) with a program code, the program code being operative for performing one of the methods when the computer program product runs on a computer. The program code may, for example, be stored on a machine-readable carrier. Other embodiments comprise the computer program for performing one of the methods described herein, stored on a machine-readable carrier. In other words, an embodiment of the present invention is, therefore, a computer program having a program code for performing one of the methods described herein, when the computer program runs on a computer.

A further embodiment of the invention provides a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention provides a data stream or a sequence of signals representing the computer program for performing one of the methods described herein. The data stream or the sequence of signals may, for example, be configured to be transferred via a data communication connection, for example, via the internet.

A further embodiment of the invention provides a processing means, for example, a computer or a programmable logic device, configured to, or adapted to, perform one of the methods described herein.

A further embodiment of the invention provides a computer having installed thereon the computer program for performing one of the methods described herein.

A further embodiment of the invention provides an apparatus or a system configured to transfer (e.g., electronically or optically) a computer program for performing one of the methods described herein to a receiver. The receiver may, for example, be a computer, a mobile device, a memory device, or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

### REFERENCE SIGNS

- 1: user
- 2: article of footwear
- 2a: sensor module
- 3: electronic device
- 4: cloud platform
- 101-103: method steps

## Claims

1. A computer-implemented athletic performance monitoring method (100), the method comprising:
(a) receiving (101) input data including biomechanical measurement data captured by a sensor module (2a) of an article of footwear (2) worn by a user (1) engaged in an athletic activity;
(b) processing (102), preferably automatically processing, the input data using artificial intelligence to generate athletic performance information, wherein the processing comprises:
(i) generating (102a) a prompt for a generative language model based at least in part on the input data;
(ii) prompting (102b) the generative language model using the prompt to obtain a reply from the generative language model; and
(iii) generating (102c) the athletic performance information based at least in part on the reply from the generative language model; and
(c) causing displaying (103) the athletic performance information to the user (1) on a display screen of an electronic device (3) of the user (1).

2. The computer-implemented method of claim 1, wherein the generative language model is a large language model (LLM) trained on a text corpus comprising general athletic performance knowledge.

3. The computer-implemented method of any one of the preceding claims, wherein the biomechanical measurement data includes plantar foot pressure distribution data and/or 3D pressure distribution data and/or gait data and/or posture data.

4. The computer-implemented method of any one of the preceding claims, wherein the input data includes external data received from at least one source other than the article of footwear (2), wherein the external data comprises one or more of: long-term athlete performance data, such as training intensity, recovery periods, and/or physical responses over time; secondary measurement data captured by a second sensor module, preferably of an equipment, a wearable device, and/or a sensor embedded in a garment worn by the individual; diet data; sleep pattern data; stress level data.

5. The computer-implemented method of any one of the preceding claims, wherein the athletic performance information comprises intra-activity athletic performance information; and wherein the step of displaying the athletic performance information comprises displaying the intra-activity athletic performance information while the individual performs the athletic activity.

6. The computer-implemented method of any one of the preceding claims, wherein the athletic performance information comprises post-activity athletic performance information; and wherein the step of displaying the athletic performance information comprises displaying the post-activity athletic performance information after the individual has finished the athletic activity.

7. The computer-implemented method of any one of the preceding claims, wherein the athletic performance information comprises one or more of the following: athlete performance trend information; a predicted future athlete performance; a training program; a suggested adjustment of a training program.

8. The computer-implemented method of any one of the preceding claims, wherein the athletic performance information comprises a predictive forecasting of at least one injury risk, wherein, optionally, the athletic performance information comprises a real-time alert and/or a recommended preventive action, in particular specific exercises and/or changes in technique and/or or rest periods, being generated based on the predictive forecasting, wherein, optionally, unusual foot pressure patterns and/or asymmetrical movements and/or excessive joint stress is detected and included in the predictive forecasting.

9. The computer-implemented method of any one of the preceding claims, wherein the athletic performance information comprises rehabilitative advice to the user (1) for recovering from injuries, preferably based on the biomechanical measurement data and obtained injury information, wherein the rehabilitative advice may include tailored rehabilitation exercises and/or a blacklist of particularly hazardous exercises and/or a whitelist of recommended exercises and/or rest period recommendations, wherein, optionally, a rehabilitative progress of the user (1) is taken into account based on obtained rehabilitative progress information.

10. The computer-implemented method of any one of the preceding claims, further comprising:
generating commands, based on the step of processing, causing an adjustment of the user interaction interface, and/or causing an adjustment of the biomechanical measurement data, and/or causing an adjustment of sensor settings of the sensor module (2a).

11. The computer-implemented method of any one of the preceding claims, further comprising:
providing a chat user interface to the user (1) to interact with the generative language model, wherein the input data further comprises chat user (1) input received from the user (1) via the chat user interface.

12. The computer-implemented method of claim 11, wherein the generative language model provides interactive engagement with the user (1) through the chat user interface, in particular being capable of receiving one or more subsequent user (1) inputs, preferably in plain text format, and of providing further personalized advice and responses in response to the one or more subsequent user (1) inputs.

13. A data processing apparatus being configured to perform a method according to any one of claims 1-12.

14. An interactive fitness support system including at least one data processing apparatus and an article of footwear (2) having a sensor module (2a), and preferably at least a cloud platform (4), the system being configured to perform a method according to any one of claims 1-12, wherein, optionally, the sensor module (2a) includes one or more pressure cells and/or one or more Inertial Measurement Units and/or one or more force-sensing resistors.

15. A computer program or a computer-readable medium having stored thereon a computer program, the computer program comprising instructions which, when the program is executed by a computer, preferably by a data processing apparatus according to claim 13, more preferably by a system according to claim 14, cause the same to carry out a method of any one of claims 1 to 12.
